# EUROPEAN PATENT SPECIFICATION

(11) **EP 4 303 299 B1**
(45) Date of publication and mention of the grant of the patent: **03.06.2026**
(21) Application number: 22715117.2
(22) Date of filing: 03.03.2022
(51) Int. Cl.: C12N 5/071, B01L 3/00, C12M 3/00, C12M 1/34, C12N 5/076, A61D 19/00, C12M 1/00

(54) **PLATE FOR SELECTING SPERMATOZOA FOR INTRACYTOPLASMIC MICROINJECTION**
PLATTE ZUR SELEKTION VON SPERMATOZOEN ZUR INTRAZYTOPLASMATISCHEN MIKROINJEKTION
PLAQUE DE SÉLECTION DE SPERMATOZOÏDES POUR LA MICROINJECTION INTRACYTOPLASMATIQUE

(30) Priority: 03.03.2021 ES 202130179
(43) Date of publication of application: 10.01.2024
(73) Proprietor: Instituto Bernabeu, S.L., 03016 Alicante (ES)
(72) Inventor: BERNABEU PÉREZ, Rafael Francisco, 03016 ALICANTE (ES); TEN MORRO, Jorge, 03016 ALICANTE (ES); MARTÍ FERRI, Laura, 03016 ALICANTE (ES)
(74) Representative: Isern Patentes y Marcas S.L.
(86) International application number: PCT/ES2022/070118
(87) International publication number: WO 2022/184960

(56) References cited:
- CN-A- 105 936 890
- CN-U- 201 469 382
- WANG CAIZHU ET AL: "Cumulus oophorus complexes favor physiologic selection of spermatozoa for intracytoplasmic sperm injection", FERTILITY AND STERILITY, ELSEVIER, AMSTERDAM, NL, vol. 109, no. 5, 28 March 2018 (2018-03-28), pages 823 - 831, XP085397395, ISSN: 0015-0282, DOI: 10.1016/J.FERTNSTERT.2017.12.026
- NAKNAM WASIN ET AL: "Effect of sperm selection method by cumulus oophorus complexes and conventional sperm preparation method on sperm quality and DNA fragmentation for assisted reproduction techonology", EUROPEAN JOURNAL OF OBSTETRICS & GYNECOLOGY AND REPRODUCTIVE BIOLOGY, ELSEVIER IRELAND LTD, IE, vol. 243, 15 October 2019 (2019-10-15), pages 46 - 50, XP085910341, ISSN: 0301-2115, [retrieved on 20191015], DOI: 10.1016/J.EJOGRB.2019.10.004
- FRANKEN D R ET AL: "Can a cumulus cell complex be used to select spermatozoa for assisted reproduction?", ANDROLOGIA, BLACKWELL, BERLIN, DE, vol. 41, no. 6, 5 November 2009 (2009-11-05), pages 369 - 376, XP071589894, ISSN: 0303-4569, DOI: 10.1111/J.1439-0272.2009.00938.X
- LI KUN ET AL: "Novel distance-progesterone-combined selection approach improves human sperm quality", JOURNAL OF TRANSLATIONAL MEDICINE, vol. 16, no. 1, 20 July 2018 (2018-07-20), pages 203, XP055931313, Retrieved from the Internet <URL:https://translational-medicine.biomedcentral.com/track/pdf/10.1186/s12967-018-1575-7.pdf> DOI: 10.1186/s12967-018-1575-7

## Description

### Field of the invention

The present invention falls within the field of male fertility and relates to a plate for selecting spermatozoa, as well as a method of use for selecting spermatozoa.

### Background of the invention

Intracytoplasmic sperm microinjection (ICSI) is currently the most widely used technology in assisted reproduction centres around the world. To perform ICSI, mature oocytes and capacitated spermatozoa must be arranged in microdroplets deposited on a polystyrene plastic plate (for example, USP class VI). For commercial use, this plate must be sterile and must be embryo-tested. In general, these ICSI plates are transported to an inverted microscope and with the help of micromanipulation systems, the oocyte is held while the sperm is introduced therein.

To deposit spermatozoa on the ICSI plate, the semen sample must be previously capacitated, i.e., the semen is prepared to be used in the different assisted reproduction techniques (ART). Two sperm capacitation techniques are mainly used for this: density gradients and swim-up, the purpose of which is to obtain spermatozoa with good morphology and motility. These improvements have been translated into the development of new sperm selection techniques with the aim of selecting more competent spermatozoa to improve the results of assisted reproduction treatments (ART).

Document ES1229586U describes a device for selecting spermatozoa by applying a temperature gradient.

Document WO2018154169A1 describes a device for selecting spermatozoa according to the morphology and movement capacity thereof.

However, these devices for selecting spermatozoa do not select spermatozoa with good fertilising capacity nor genetically normal spermatozoa since, among other things, they fail to imitate the natural conditions that occur inside the female tract.

In *in vivo* conditions, the spermatozoa inside the female tract undergo a series of changes at the membrane level that are required to be able to fertilise the oocyte. By contrast, the documents of the state of the art on intracytoplasmic sperm injection (ICSI) do not take into account the natural or physiological conditions for selecting the spermatozoon, since it is introduced directly inside a mature oocyte (in Meiosis phase II, MII) with the help of a micropipette.

During natural fertilisation, the cumulus oophorus cell complex (COC) and the extracellular matrix (ECM) thereof surround the oocyte and only the spermatozoa that pass through the COC have the opportunity to reach and penetrate the zona pellucida and consequently fertilise the oocyte. If the ovulated oocyte is completely devoid of COC, it remains unfertilised.

Various studies have shown that spermatozoa capable of passing through the cumulus oophorus complex exhibit a higher capacitation pattern, better motility and morphology, and a lower rate of DNA fragmentation. Naknam Wasin et al., "Effect of sperm selection method by cumulus oophorus complexes and conventional sperm preparation method on sperm quality and DNA fragmentation for assisted reproduction technology", EUROPEAN JOURNAL OF OBSTETRICS & GYNECOLOGY AND REPRODUCTIVE BIOLOGY, vol. 243, 15 October 2019, pages 46-50, describes the selection of sperm cells using a cumulus oophorus model and a device, a plate, designed to be suitable to assay the effect of the passing of spermatozoa through cumulus oophorus cells on spermatozoa properties, The device comprises, as the only elements present in the plate, two channels, positioned centrally in the plate and spatially arranged as though the plate would not be prepared to allocate any other element; in fact, the presence of any other element(s) in the plate is not mentioned or suggested in the document. Both channels comprise: compartments A1 and A2 at one end, prepared to receive spermatozoa; compartments B1 at B2 located centrally in each channel and compartments C1 and C2 at the other end of each channel, prepared to received spermatozoa which have swam from compartments A to compartments B through compartment C. One channel is used for the analysis group, placing COCs in the central compartment so that spermatozoa located in the corresponding compartment A have to be able to transverse the COCs in order to enter the corresponding area C and are used as the study group, whereas the second channel only receives sperm washing medium in the central compartment so that the spermatozoa that swim from one end of the channel to the other merely serve as part of the experimental control group. After the required experimental time, the spermatozoa are removed from the plate, being aspirated and pooled into study and control Eppendorf tubes, respectively, to analyse the properties of the spermatozoa of each experimental group.

The scientific article of Wang Caizhu et. al., "Cumulus oophorus complexes favor physiologic selection of spermatozoa for intracytoplasmic sperm injection", FERTILIY AND STERILITY, vol. 109, no. 5, 28 March 2018, pages 823-831 (https://doi.org/10.1016/j.fertnstert.2017.12.026) describes a dish comprising areas used for spermatozoa selection where COC cells are used in the physiological selection of spermatozoa for intracytoplasmic sperm injection (ICSI). The dish comprises 2 channels and a small area P for depositing PVP means for cleaning and filling the plate areas. Each of said channels comprise, respectively, areas A1 and A2 at one end of the channel, which were used to place the prepared spermatozoa. One channel comprises an area B1, in the middle of the channel, which was filled with cumulus oophorus cells (COC) and medium, whereas the other channel comprises, also in the middle, an area B2, which was filled with medium only. The sperm is expected to swim from areas A through areas B to areas C (C1 and C2) placed at the other end of each channel. Only the sperm that can pass through the COCs (the sperm swimming on the channel containing area B1) will reach area C of that channel (area C1), being selected as the study group, whereas the sperm reaching area C2 was selected as the control group. Said article does not describe the shape or dimensions of the channels or the mentioned areas on the plate are like.

Wang Caizhu's dish is designed to be suitable to assay the effect of the passing of spermatozoa through the cumulus oophorus on spermatozoa properties, not to have a plate usable in fertilization clinics. It does not mention or suggest the presence in the dish of an area where intracytoplasmic sperm microinjection (ICSI) can be performed. As indicated in said document, this step (ICSI) is carried out by extracting the spermatozoa that reach the area C by means of an ICSI injection needle, which are subsequently transferred to another conventional ICSI dish for injection into the ovum. This design slows down the intracytoplasmic microinjection process and increases the probabilities of contamination of the sperm sample.

Chinese Patent Application CN105936890A describes a method to screen sperm quality by making spermatozoa to traverse cumulus oophorus cells, with the aim of using spermatozoa subsequently for fertilization. The application includes the drawing of a device very similar to the one described by Wang Caizhu et. al. (see complete reference supra), but showing only one channel (also with a compartment for loading spermatozoa at one end, a central compartment for COCs and a collection compartment at the other end to receive the spermatozoa from the first compartment having traverse the COCs) and also showing a small oval, labelled as PVP, which might represent a small hole, parallel to the more elongated dimension of the channel, with no indicated function. The sperm arriving at the final collection compartment improves ovum fertilization rate, blastocysts forming rate and ovum utilization rate.

The devices described by Naknam Wasin et al. (see complete reference supra), Wang Caizhu et al. (see complete reference supra) and in application CN105936890A are presented as devices for spermatozoa selection or quality improvement. The specific dimensions, relative arrangement and relative distances and proportions of the elements of the devices (channel compartments, channel conduits joining compartments, the whole channel portion of the device and, in the case of CN105936890A and the Wang Caizhu et al. article, also the PVP area) are not mentioned in any of said three documents, although, according to the drawings, the central compartments for COCs have a horizontal section which is circular, oval or elliptical and additionally, smaller than the sperm loading compartments in all three documents. In the particular case of the Naknam Wasin et al. article, the minimum capacities of the central compartment for COCs and the loading compartment can be deduced, since it is specified that 40 µl of sperm washing medium are added to the central compartments and 20 µl of spermatozoa to the compartments wherefrom they swim along the channels.

Franken D R et al. "Can cumulus cell complex be used to select spermatozoa for assisted reproduction?", ANDROLOGIA, vol. 41, no. 6, 5 November 2009, pages 369-376, discloses a device for the selection of sperm cells. A cumulus oophorus model in a capillary (length ca. 10 mm) is used for sperm selection. Many dimensions of the device elements are not defined and allow much variability.

Li Kun et al., "Novel distance progesterone-combined selection approach improves human sperm quality", JOURNAL OF TRANSLATIONAL MEDICINE, vol. 16, no. 1, 20 July 2018, page 203ff, describes a device which tries to mimic the female human reproductive system for the selection of sperm cells, where long distance channels are combined with a progesterone gradient and human tube fluid media.

Chinese utility model CN201469382U describes a culture dish for sperm selection comprising grooves in a length range of 2-10 cm where selection by cumulus oophorus penetration is not considered.

Therefore, there is a need to solve the aforementioned problems and to provide a suitable plate to carry out selection and microinjection and which also enables a good spermatozoa selection.

### Brief description of the drawings

As a complement to the description, said description is accompanied by a set of drawings wherein, in Figures 1 to 4, the shape and relative dimensions of the compartments is only illustrative and not exactly corresponding to the claimed invention, which drawings represent the following:
**Figure 1****:** Upper front view of the selection plate, wherein the microinjection portion (1) or area (1) of diameter (a) and two lanes (2a and 2b), can be seen.
**Figure 2****:** Upper front view of a lane, wherein the loading compartment for the spermatozoa sample (3), the compartment (4) where the cumulus oophorus cells are placed, the collection compartment (5) and the conduit (6) that joins the 3 compartments can be seen.
**Figure 3****:** In the upper portion, the upper front view of a lane (2) can be seen with the signs indicating the cross-sectional views of the compartments (3) and (5) shown in the lower portion; a portion of the conduit (6) can also be seen.
**Figure 4****:** In the upper portion, the upper front view of a lane (2) can be seen with the sign indicating the longitudinal cross-sectional view of the same in the lower portion; the compartments (3), (4) and (5) and the conduit (6) can also be seen.
**Figure 5****:** Profile view of the selection plate comprising a protrusion (7) that surrounds it.
**Figure 6****:** Upper front view, parallel to the plane of the surface of the plate wherein an embodiment of a lane (2) of the plate of the invention is described, comprising the loading compartment for the sperm sample (3), the compartment (4) where the cumulus oophorus cells are placed, the collection compartment (5) and the conduit (6) that joins the 3 compartments, wherein the compartment (4) has a size and amplitude greater than the compartments (3) and (5).

### Detailed description of the invention

As used herein, the term "oocyte" refers to a female gamete, which may be immature (prophase I, PI, metaphase I, MI) or mature (metaphase II, Mll). Said mature oocyte is in a condition to be fertilised by a spermatozoon. Preferably, the spermatozoa used in the method of the invention are mammalian spermatozoa. More preferably, the spermatozoa are selected from a list consisting of humans, cattle, and companion animals (including, but not limited to, canines and felines). Cattle sperm can be bovine, porcine and/or ovine. Companion animal sperm can be canine and feline.

The term "cumulus oophorus cells" relates to cells in the developing ovarian follicles that are in direct or close proximity to an oocyte. Cumulus cells are granulosa cells that surround the oocyte both in the ovarian follicle and after ovulation.

As used herein, the term "sperm" or "spermatozoon" relates to a male reproductive gamete. A uniflagellar sperm cell that is motile or non-motile is also known as a spermatozoon.

The terms "enlargement" and "compartment" are synonymous and are used interchangeably.

The first aspect of the invention relates to a plate for the physiological selection of spermatozoa for ICSI comprising at least two portions, wherein one portion comprises at least one lane (2), each lane having three compartments (3, 4, 5) wherein:
- the compartment (3) is adapted to receive a processed semen sample;
- the compartment (4) is adapted to deposit cumulus oophorus cells;
- the compartment (5) is adapted to host and recover spermatozoa from the processed semen sample;
the compartments (3) and (5) being located at the ends of the lane (2), the compartment (4) being located between the compartments (3) and (5) and all of them connected by means of a conduit (6);
characterized in that
- the lane (2) is configured as indentations or grooves in the plate and its length is in a range between 20 and 30 mm;
- the compartments (3), (4) and (5) and the conduit (6) are formed by means of an indentation or groove in the plate;
- the plate comprises a microinjection area (1) located in the opposite portion wherein the lane (2) is located, said microinjection area (1) being suitable for depositing oocytes and performing the microinjection of the spermatozoa selected from the compartment (5) in the deposited oocytes, wherein the microinjection area (1) has an area between 32-8 cm²;
- the section defined by the plane of the compartments (3) and (5), parallel to the plane of the surface of the plate, has a width or diameter less than the width or diameter of the section defined by the plane of the compartment (4) parallel to the plane of the surface of the plate, and which is in a range between 2.5-4 mm, and
- the compartment (4) has a larger area than the compartments (3) and (5) and the section defined by the plane of the compartment (4), parallel to the plane of the surface of the plate, has a diameter or width between 2.5-5 mm;
- the conduit (6) and the compartments (3), (4) and (5) have a depth (6a), (3a), (4a), (5a) respectively, in a range between 0.4-0.9 mm;
- the compartment (4) is located at the same distance from the end compartments (3) and (5);
- the section defined by the plane of the conduit (6), parallel to the plane of the surface of the plate, has a width or diameter in a range between 1 and 2 mm;
   and
- the plate has a height or depth between 0.5-20 mm and the depth of the lane (2) is in a range between 20-80% of the height or depth of the plate.

The conduit (6) has the function of helping to conduct fluids from one place to another, preferably between the compartments (3), (4) and (5).

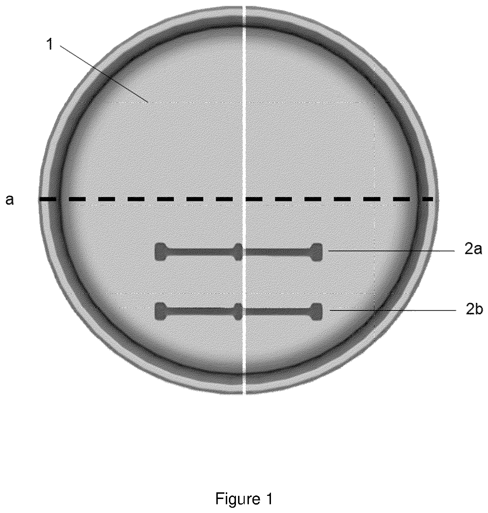

The term "plate portion" refers to one of the 2 portions or halves divided by means of the diameter or longitudinal axis (a) of the selection plate (Figure 1). The portions do not need to have to have exactly the same area. The selection plate of the invention has the advantage that it comprises 2 portions, at least one lane (2) is located in one of the portions, and the microinjection area (1) (ICSI) is located in the other portion, opposite to the area or portion where the lane (2) is located.

The lane (2) is configured as an indentation or groove in the plate. This allows safe handling of the plate and the biological material, both the semen sample and the cumulus oophorus cell sample, without loss, spillage, or contamination between them. Consequently, a better yield is obtained in the number of spermatozoa that reach the lane (5). Preferably, the lane (2) has a circular, elliptical or oval shape, which enables a better use of the semen sample and the cumulus oophorus cell sample, reducing losses of said samples in irregular portions of the lane (2). Consequently, a better yield is obtained in the number of spermatozoa that reach the lane (5). In the same way, the compartments (3, 4 and 5) as well as the conduit (6) are also formed by means of an indentation or groove in said plate. This arrangement makes it easier to work on the plate, avoiding the interaction of the semen sample with the cumulus oophorus cells, providing a better selection and reducing and/or eliminating the contamination of the different compartments.

The plate comprises a microinjection area or portion (1) (ICSI), opposite to the area or portion where the lane (2) is located, as Figure 1 shows. This microinjection portion (1) or area (1) is suitable and large enough to deposit the oocytes and perform the microinjection (ICSI) of the selected spermatozoa in the other portion that comprises the lane (2). The plate of the invention has the advantage that it is adapted to carry out the spermatozoa selection. This design significantly reduces the chances of contamination or loss of the selected sperm sample.

In a preferred embodiment of the plate of the invention, the microinjection area or portion (1) (ICSI), has an area between 32-8 cm². Said size is suitable for depositing different sizes of oocyte samples.

The microinjection area (1) is adapted to deposit the oocytes, preferably as microdroplets, at least 2 microdroplets containing oocytes, more preferably no more than 6 microdroplets.

In a preferred embodiment, the microinjection area (1) comprises an indentation or groove to deposit the oocytes.

The arrangement of the lane (2) or lanes and the microinjection area (1) in the plate avoids contamination of the cumulus oophorus cells on the oocytes deposited in the opposite portion. Therefore, the plate of the invention provides a better selection and reduces and/or eliminates contamination between the different compartments and portions.

The lane (2) is configured to receive the semen sample, said sample being incorporated into the compartment (3), where the compartment (3) is located at one end. The lane (2) is also configured to deposit the oophorus cumulus cells in the compartment (4), located in the center, and to recover the spermatozoa that have reached the compartment (5). The compartment (5) is located at one end of the lane; therefore, the lane (2) is configured so that the spermatozoa reach the compartment (5), located at the opposite end to the compartment (3).

In a preferred embodiment, the selection plate has 2 lanes (2a) and (2b), the lane (2a) or (2b) being located parallel to the other lane (2a) or (2b) at a separation distance of at least 3 mm and both lanes (2a) and (2b) being located in the same portion of the plate. Said portion being the lower portion, as seen in Figure 1, of one of the 2 portions divided by means of the diameter or longitudinal axis (a) of the selection plate.

According to that preferred embodiment of the plate of the invention, the plate comprises 2 parallel lanes (2a) and (2b) located at a separation distance of at least 3 mm, to avoid contamination between the compartments of two different lanes, preferably to avoid contamination of the biological material (cumulus oophorus cells) from the compartment (4) in the compartment (3) (semen sample). Preferably the separation distance is at least 5 mm, being more preferably 7 mm in a preferred embodiment. The separation distance between lanes is measured by taking the center of each lane as a reference point. The lanes must not be in contact with each other in any case.

The selection plate can have any geometry; in a preferred embodiment the selection plate is circular or oval, as it facilitates gripping and handling. For the purposes of better clarity, in the event that the geometry of the plate is not regular, the diameter or longitudinal axis of the plate to be considered to identify each portion of the same is the one that connects the 2 most distal ends.

The selection plate can be reused or can be a disposable article configured for a single use. In a preferred embodiment, the selection plate is made of polystyrene, more preferably the polystyrene has a density from 1-1.1 g/CC, tensile strength from 35-50 MPa and/or a modulus of elasticity from 2.7-3.5 GPa.

The length of the lane (2) is in a range between 20 and 30 mm. In a preferred embodiment, the lanes (2a) and (2b) have the same length.

In another preferred embodiment of the plate of the invention, the distance between the longitudinal axis or diameter of the selection plate (a) and the lane closest thereto is at least 3 mm, preferably 5 mm, more preferably 7 mm.

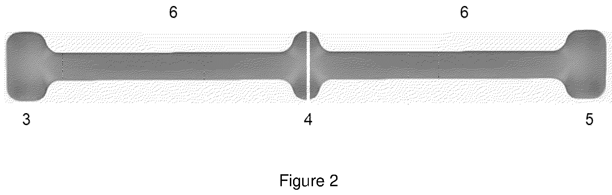

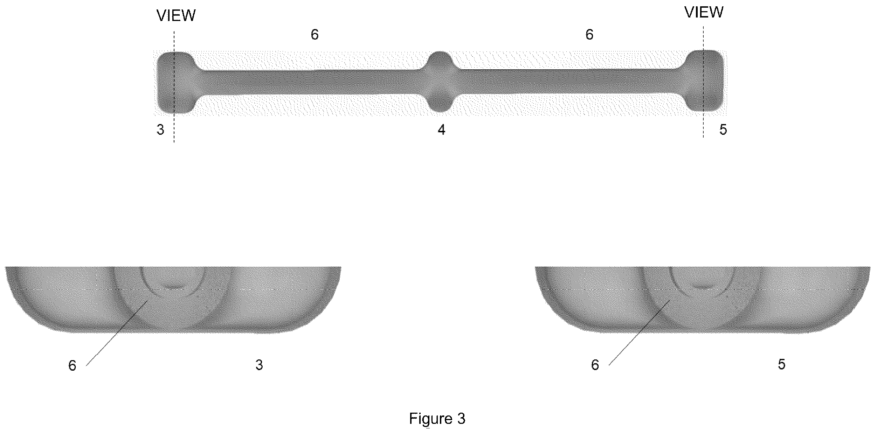

The lane of the selection plate comprises at least the compartments (3) (4), (5) connected to each other by a conduit (6), as described in Figures 2 and 3.

The compartment (3) is suitable or configured to receive a processed semen sample, i.e., where the spermatozoa are deposited and is located at the ends of the lane (2). The compartment (3) can also be referred to as loading compartment.

The compartment (4) is configured to deposit the cumulus oophorus cells and is located between the end compartments (3) and (5).

The compartment (5) corresponds to the recovery compartment, i.e., to the final area where the spermatozoa that have managed to pass through the compartment (4) will reach. The spermatozoa contained in the compartment (5) may be recovered by means of extraction means, preferably by means of a micropipette, to be injected at subsequent steps inside the oocyte.

The section of the compartments (3), (4) and (5) is defined by a plane parallel to the surface of the plate. Said section can have a circular, oval, elliptical, square or rectangular shape

The term "width" corresponds to the greatest width or length of this section of the compartments when they do not have a circular or circumferential shape, i.e., when the compartments have, for example, a rectangular, square, elliptical or oval shape.

The term "diameter" corresponds to the width of said section when the compartment is circular in shape.

The width or diameter of the section of the compartments (3) and (5) parallel to the plane of the surface of the plate is less than the width or diameter of the section defined by the plane of the compartment (4) parallel to the plane of the surface of the plate and is comprised in a range between 2.5-4 mm.

The width or diameter of the section of the compartment (4), parallel to the plane of the surface of the plate, also referred to as (4b), is comprised in a range between 2.5-5 mm, simultaneously providing good efficiency as well as good performance. When the width or diameter (4b) is less than 2.2 mm, the performance decreases. Likewise, when the width or diameter (4b) is more than 5 mm, the efficiency decreases. The dimensions of the compartment (4) also make it suitable for depositing enough cumulus oophorus cells, since if the dimensions are smaller, not enough oophorus cells can be deposited to carry out the spermatozoa selection.

The width or diameter of the section of the compartment (5), parallel to the plane of the surface of the plate, also referred to as (5b), is comprised in a range between 2.5-4 mm. The dimensions of the compartment (5) and the position thereof in the lane make it suitable for receiving the spermatozoa that have passed through the compartment (4) and be able to recover them using extraction means, preferably with a micropipette, to be injected in subsequent steps inside the oocyte.

The width or diameter of the section of the compartment (3), parallel to the plane of the surface of the plate, also referred to as (3b), is comprised in a range between 2.5-4 mm, more preferably between 2.5-3 mm. The dimensions of the compartment (3) make it suitable for receiving and depositing the processed semen sample, as well as for optimizing performance in relation to the number of spermatozoa that reach the compartment (5).

The end compartments (3) and (5) are identical or different; preferably they are identical and can adopt any polygonal shape.

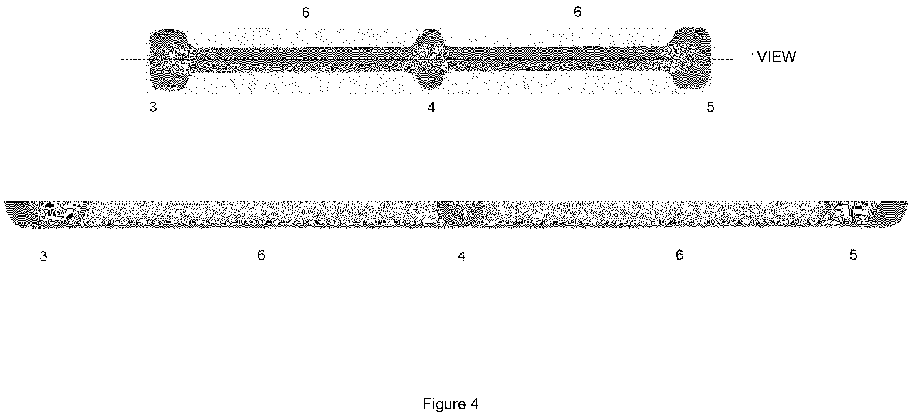

The central compartment (4) is located between the end compartments (3) and (5) at the same distance from the end compartments (3) and (5) (Figure 4).

In another preferred embodiment, the selection plate of the invention further comprises a cover that covers the entire surface thereof. In this way, the plate of the first aspect has the advantage that it prevents the evaporation of the liquids in the semen sample, in the cumulus oophorus cells and during the step of spermatozoa selection, preserving the quality and concentrations of both the semen sample and cumulus cells and improving performance in relation to the number of spermatozoa recovered in the compartment (5) and the selection of appropriate spermatozoa in terms of the morphology and motility thereof, for subsequent microinjection.

The depth of the lane (2) corresponds to the depth (6a) of the conduit (6) and the depth of the compartments (4), (3) and (5), therefore (3a), (4a) and (5a) respectively.

The depth of the lane (2), the conduit (6) and the compartments (4), (3) and (5) is the distance between the surface of the plate and the bottom of the lane (2).

The depth of the conduit (6) and the depth of the compartments (4), (3) and (5) are in a range between 0.4-0.9 mm, more preferably between 0.5-0.7 mm. The width or amplitude or diameter of the section defined by the plane of the conduit (6), parallel to the plane of the surface of the plate, also referred to as (6b), is in a range between 1-2 mm, more preferably between 1.2-1.6 mm. These ranges allow working with different types of semen samples, in terms of the quality thereof, thus improving the versatility and applicability of the plate for use with semen samples from different subjects. In addition, they allow for correct heat transfer to the samples. If this depth of the conduit is less than 0.2 mm, adequate heat transfer to the plate to carry out the spermatozoa selection does not occur.

Likewise, in the case of the compartment (4) they allow working with a different sample size of the cumulus cells, adapting to different types of patients who may need a greater number of cumulus cells to carry out the spermatozoa selection.

The plate for selecting spermatozoa of the first aspect of the invention has a height or depth between 0.5-20 mm. The depth of the lane (2), which includes the depth of the conduit (6) and the compartments (4), (3) and (5), is in a range between 20-80% of the height or depth of the plate, more preferably between 40-60% of the height or thickness of the plate. In this way, correct heat transfer from the plate to the samples for performing the spermatozoa selection is ensured.

The compartment (4) has a larger area than the compartments (3) and (5). More preferably, the width or diameter (4b) of the compartment (4) is greater than in the compartments (3) and (5) and is in a range between 2.5-5 mm and the width or diameter of the compartments (3) and (5) is in a range between 2.2-3 mm. In this particular narrower combination of features of the invention, a better selection is obtained and contamination between the different compartments and portions is reduced and/or eliminated. In the case of the compartment (4), these dimensions allow working with a different sample size of cumulus cells, adapting to different types of patients who may need a greater number of cumulus cells to carry out the spermatozoa selection.

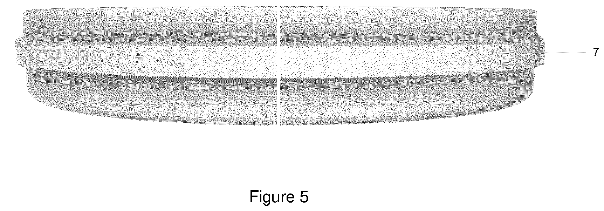

In another preferred embodiment of the plate of the invention, the plate further comprises a protrusion (7) that surrounds the contour, as seen in Figure 5. Said protrusion (7) facilitates gripping and handling of the plate. In addition, it enables gas exchange between the inside of the plate and the outside by leaving a small space between the portion where the lane (2) is located and the cover, which improves the extraction of spermatozoa from the compartment (5), helping to maintain a stable pH, particularly when a buffered medium is not used, reducing fertilisation failures.

In a particularly preferred embodiment of the plate of the invention:
- the lane (2) has a length of 22 ± 0.2 mm,
- the section defined by the plane of the compartments (3) and (5), parallel to the plane of the surface of the plate has a width of 2.5 mm and a length of 1.48 mm,
- the section defined by the plane of the compartment (4), parallel to the plane of the surface of the plate has a width of 5 mm and a length of 3 mm;
- the section defined by the plane of the conduit (6), parallel to the plane of the surface of the plate has a width or diameter of 1 mm.

The second aspect of the invention relates to a method for the physiological selection of spermatozoa comprising the use of the selection plate according to the first aspect of the invention from a processed semen sample. Said method provides good and efficient spermatozoa selection, as well as a good yield. The plate and the method of the invention are suitable for selecting spermatozoa for fertilising oocytes and/or ovules from mature women, preferably in women between the ages of 34 and 44, more preferably in women between 34-40 years.

The second aspect of the invention relates to a method for the physiological selection of spermatozoa comprising the use of the plate of the invention and comprising at least the following steps:
a) providing cumulus oophorus cells in the compartment (4) of the plate of the invention,
b) providing a processed semen sample in the compartment (3) of the plate of the invention,
c) incubating the plate at a temperature in a range between 36-38°C, for a period of time comprised between 10 min and 3 hours, and
d) extracting the spermatozoa that are in the recovery compartment (5).

The method of the invention is not carried out inside the human or animal body. In the context of the present invention, the term "processed semen sample" refers to a semen sample from which seminal plasma has been removed; therefore, said semen sample does not comprise disabling substances, prostaglandins and/or leukocytes. The processed semen sample can be obtained following processes of the common knowledge of the state of the art, known to those skilled in the art. The processed semen sample can be obtained through a process comprising: performing a seminogram of said sample, followed by performing a procedure for the treatment of the semen sample, by density gradient or swim up. Once the density gradient is finished, the processed semen sample is adjusted to obtain the semen sample with a concentration of spermatozoa/ml in a specific sperm washing medium, such as the medium known in the state of the art as sperm-washing, which concentration, for the purposes of the present invention, can be in a range between 5 and 20 million spermatozoa/ml, preferably 10 million spermatozoa/ml. In an embodiment of the method of the invention, the processed semen sample is added in step b) in an amount between 1 and 10 µl and having a concentration between 5 and 20 million spermatozoa/ml. In a preferred embodiment the processed semen sample is added in step b) in a range between 2-5 µl and has a concentration of 10 million spermatozoa/ml.

The cumulus oophorus cells of step a) are previously obtained from the cumulus-oocyte complexes. Cumulus-oocyte complexes are obtained using follicular puncture. With the use of syringes, part of the cumuli is removed and stored for subsequent use. Preferably, cumuli with a loose appearance will be selected. Said morphological characteristic can be determined by a person skilled in the art by using a magnifying glass. Next, decumulation is carried out by the action of the enzyme hyaluronidase, the main component of the ECM (extracellular matrix), which is synthesised by the cumulus cells after the surge of LH (luteinising hormone), according to a standard protocol in the state of the art. Briefly, the decumulation protocol comprises the elimination of cumulus cells using hyaluronidase. The cumulus-oocyte complexes must not be in contact with hyaluronidase for more than 30 seconds. During this process, the complexes are manipulated with capillaries of different diameters that help to eliminate, by mechanical action, the granulosa cells that remain more adhered to the oocytes. This process is performed outside the incubator for a period of 10 minutes. Preferably, said period of time does not extend beyond 10 min. After this procedure, the MII available for microinjection and the cumulus oophorus cells for spermatozoa selection are obtained.

In a preferred embodiment of the method of the invention, the processed semen sample is human.

In the method of the invention, the incubation process of step c) is carried out by using suitable means to provide a temperature to the plate in a range between 36-38°C.

Preferably, suitable means for providing a temperature are selected from the list consisting of a stove, an incubator, a heat lamp or a convection oven.

During the incubation process, the spermatozoa deposited in the compartment (3) migrate towards the cumulus oophorus cells previously deposited in the compartment (4) through the channel (2), preferably through 2 channels (2a) and (2b), and only those who manage to pass through them will pass to the compartment (5), located at the end of the lane.

In a preferred embodiment of the method of the invention, the incubation period of step c) takes place in a period of time comprised in a range between 10 min and 3 hours; in a preferred embodiment between 30 min and 90 min, more preferably between 45 min and 75 min, and in a more preferred embodiment 60 min, the latter optionally combined with providing the processed semen sample of step b) in amount between 2-5 µl and having a concentration of 10 million spermatozoa/ml. These time ranges used improve the number of spermatozoa found in the compartment (5) as well as the quality of the spermatozoa, which will be more capacitated for the subsequent intracytoplasmic microinjection of the oocyte.

In a particular embodiment, the selection method of the second invention comprises an additional step e), prior to step a), comprising the addition of a sperm-specific washing medium to the lane (2), preferably the washing medium is the sperm-wash medium. This step improves the number of blastocysts obtained and the quality thereof, as well as spermatozoa selection.

The third aspect of the present invention relates to the use of the selection plate of the invention in a method for selecting spermatozoa.

### Example of plate according to the invention, a similar plate not covered by the invention and method of the invention for the physiological selection of spermatozoa

Evaluation of the effectiveness of the invention and the method of the invention for the selection of spermatozoa for ICSI therapy.

A plate with 1 single lane (2) was used which corresponded to the one in Figure 4, which departed from the present invention in the broadness of the possible width range of compartment (3) and the fact that the area of compartment (4) was not larger than that of compartments (3) and (5). Width of the compartment (3): 2-4 mm, width of the compartment (4): 3-4 mm and width of the compartment (5): 4 mm. Width of the conduit (6) 1.5 mm. Polystyrene plate.

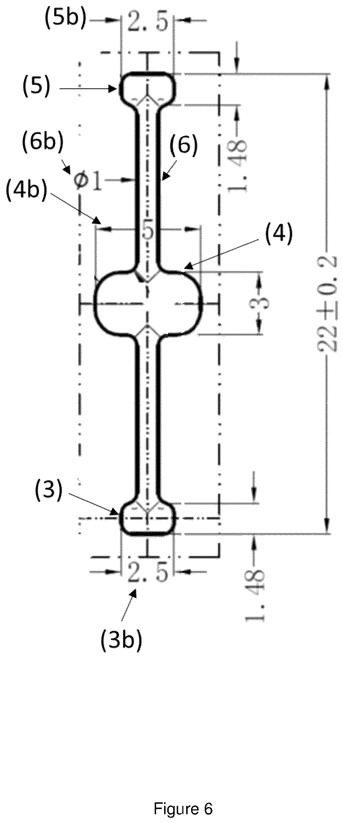

A plate according to the present invention corresponding to Figure 6 was also tested and provided very good selection results.

### Method for selecting spermatozoa

A cumulus oophorus sample was provided, previously obtained from cumulus-oocyte complexes as described above together with the claimed embodiments of the method of the invention. Said sample was deposited in the compartment (4) of the plate.

Next, 4 µl of a processed semen sample was provided and deposited in the compartment (3) of the plate.

Next, the plate was incubated at a temperature in a range between 37°C, for a period of time comprised between 60 min. Once the incubation process was completed, the spermatozoa that managed to reach the recovery compartment (5) were observed. Finally, said spermatozoa were extracted from the compartment 5 with a micropipette to be subsequently injected into the oocytes located in the microdroplets in the microinjection portion (1) or area.

For the study, the effect on embryonic development up to the blastocyst stage and the quality thereof was evaluated.

For this, a prospective randomised study was performed wherein 995 oocytes from 90 patients who underwent IVF treatment were included. Two groups were generated: a control group, 498 oocytes, which were microinjected with conventionally selected sperm, and a study group (n=497) which were microinjected with sperm selected by means of granulosa cells in the plate. All semen samples were processed using density gradients.

Valid cases for blastocyst formation rate and embryo quality=757 fertilised oocytes. The study group with the plate represented by Figure 4 and the method of the invention was compared with the control group. The study group was prepared with granulosa cells. Sperm preparation in all cases was performed using conventional methods (density gradients).

**Table 1: Results of the study.**

| | Control group | Study group |
|---|---|---|
| Fertilisation (2PN+2CP) | 74.0% | 78.7% |
| Blastocyst formation rate | 60.1% | 70.9% |
| Good quality blastocysts (A+B) | 38.5% | 49.6% |
| Poor quality blastocysts (C+D) | 61.5% | 50.4% |

There were more fertilised oocytes in the plate of the study group, represented by Figure 4, with the sperm selected from that plate than in those of the control group. This result is especially good for oocytes from women of mature ages between 34-44 years, more preferably between 34-40 years. The mean age of the study was 37 years.

The results with the plate represented by Figure 4 and also with the method of the invention showed significant improvements in relation to the blastocyst formation rate, the percentage of good quality blastocysts and fertilisation, which were higher with the plate of the invention compared to the group control, as seen in Table 1.

## Claims

1. A plate for the physiological selection of spermatozoa for ICSI comprising at least two portions, wherein one portion comprises at least one lane (2), each lane having three compartments (3, 4, 5) wherein:
- the compartment (3) is adapted to receive a processed semen sample;
- the compartment (4) is adapted to deposit cumulus oophorus cells;
- the compartment (5) is adapted to host and recover spermatozoa from the processed semen sample;
the compartments (3) and (5) being located at the ends of the lane (2), the compartment (4) being located between the end compartments (3) and (5) and all of them connected by means of a conduit (6);
**characterized in that**
- the lane (2) is configured as indentations or grooves in the plate and its length is in a range between 20 and 30 mm;
- the compartments (3), (4) and (5) and the conduit (6) are formed by means of an indentation or groove in the plate;
- the plate comprises a microinjection area (1) located in the opposite portion wherein the lane (2) is located, said microinjection area (1) being suitable for depositing oocytes and performing the microinjection of the spermatozoa selected from the compartment (5) in the deposited oocytes, wherein the microinjection area (1) has an area between 32-8 cm²;
- the section defined by the plane of the compartments (3) and (5), parallel to the plane of the surface of the plate, has a width or diameter less than the width or diameter of the section defined by the plane of the compartment (4) parallel to the plane of the surface of the plate, and which is in a range between 2.5-4 mm, and
- the compartment (4) has a larger area than the compartments (3) and (5) and the section defined by the plane of the compartment (4), parallel to the plane of the surface of the plate, has a diameter or width between 2.5-5 mm;
- the conduit (6) and the compartments (3), (4) and (5) have a depth (6a), (3a), (4a), (5a) respectively, in a range between 0.4-0.9 mm;
- the compartment (4) is located at the same distance from the end compartments (3) and (5);
- the section defined by the plane of the conduit (6), parallel to the plane of the surface of the plate (1), has a width or diameter in a range between 1 and 2 mm;
and
- the plate has a height or depth between 0.5-20 mm and the depth of the lane (2) is in a range between 20-80% of the height or depth of the plate.

2. The plate according to the preceding claim, comprising at least 2 lanes (2b) and (2a) located parallel to each other at a separation distance of at least 3 mm and in the lower portion of the 2 portions of the plate divided by means of the diameter or longitudinal axis (a) of the plate, wherein the lanes (2a) and (2b) have the same length.

3. The plate according to claim 2, wherein the separation distance between the lanes is 7 mm.

4. The plate according to any one of the preceding claims, the distance between the longitudinal axis or diameter of the selection plate (a) and the lane closest thereto is 7 mm.

5. The plate according to any one of the preceding claims, wherein the microinjection area (1) comprises an indentation or groove to deposit the oocytes.

6. The plate according to any one of the preceding claims, which plate is circular or oval,
and/or
further comprises a protrusion (7) that surrounds the contour.

7. The plate according to any one of the preceding claims, wherein
- the lane (2) has a length of 22 ± 0.2 mm,
- the section defined by the plane of the compartments (3) and (5), parallel to the plane of the surface of the plate (1), has a width of 2.5 mm and a length of 1.48 mm,
- the section defined by the plane of the compartment (4), parallel to the plane of the surface of the plate (1), has a width of 5 mm and a length of 3 mm;
- the section defined by the plane of the conduit (6), parallel to the plane of the surface of the plate (1), has a width or diameter of 1 mm.

8. A method for the physiological selection of spermatozoa comprising the use of the selection plate according to any of claims 1-7, for a processed semen sample. . comprising the following steps:
a) providing cumulus oophorus cells in the compartment (4) of the selection plate according to any of claims 1-7,
b) providing a processed semen sample in the compartment (3) of the selection plate according to any of claims 1-7,
c) incubating the plate obtained in step a) and/or b) at a temperature in a range between 36-38°C, for a period of time comprised between 10 min and 3 hours, and
d) extracting the spermatozoa that are in the compartment (5).

9. The method according to the preceding claim , wherein the processed semen sample of step b) is provided in an amount between 1 and 10 µl and having a concentration between 5 and 20 million spermatozoa/ml.

10. The method according to any one of the preceding claims 8-9, wherein the incubation period of step c) is comprised in a range between 30 min and 90 min.

11. The method according to claim 8, wherein:
i) the processed semen sample of step b) is provided in an amount between 2-5 µl and having a concentration of 10 million spermatozoa/ml, and/or
ii) the incubation period of step c) is 60 minutes.

12. The method according to any one of the preceding claims, wherein the semen sample is human.

13. A use of the selection plate according to any of claims 1-7 in a method for selecting spermatozoa.

## Patentansprüche

1. Platte zur physiologischen Selektion von Spermatozoen für ICSI, umfassend mindestens zwei Abschnitte, wobei ein Abschnitt mindestens eine Spur (2) umfasst, wobei jede Spur drei Fächer (3, 4, 5) aufweist, wobei:
- das Fach (3) dazu angepasst ist, eine verarbeitete Samenprobe aufzunehmen;
- das Fach (4) dazu angepasst ist, Cumulus-oophorus-Zellen abzulegen;
- das Fach (5) dazu ausgelegt ist, Spermatozoen aus der verarbeiteten Samenprobe zu beherbergen und wiederzugewinnen;
wobei sich die Fächer (3) und (5) an den Enden der Spur (2) befinden, während sich das Fach (4) zwischen den Endfächern (3) und (5) befindet und alle durch eine Leitung (6) miteinander verbunden sind;
**dadurch gekennzeichnet, dass**
- die Spur (2) als Vertiefungen oder Rillen in der Platte konfiguriert ist und ihre Länge im Bereich zwischen 20 und 30 mm liegt;
- die Fächer (3), (4) und (5) und die Leitung (6) mittels einer Vertiefung oder Rille in der Platte gebildet sind;
- die Platte einen Mikroinjektionsbereich (1) umfasst, der sich am gegenüberliegenden Abschnitt befindet, wo sich die Spur (2) befindet, wobei der Mikroinjektionsbereich (1) zum Ablegen von Oozyten und zum Durchführen der Mikroinjektion der aus dem Fach (5) selektierten Spermatozoen in die abgelegten Oozyten geeignet ist, wobei der Mikroinjektionsbereich (1) eine Fläche zwischen 32-8 cm² aufweist;
- der Querschnitt, der durch die Ebene der Fächer (3) und (5) parallel zur Ebene der Oberfläche der Platte definiert wird, eine Breite oder einen Durchmesser aufweist, der kleiner ist als die Breite oder der Durchmesser des Querschnitts, der durch die Ebene des Fachs (4) parallel zur Ebene der Oberfläche der Platte definiert wird, und der in einem Bereich zwischen 2,5-4 mm liegt, und
- das Fach (4) eine größere Fläche aufweist als die Fächer (3) und (5) und der Querschnitt, der durch die Ebene des Fachs (4) parallel zur Ebene der Oberfläche der Platte definiert wird, einen Durchmesser oder eine Breite zwischen 2,5-5 mm aufweist;
- die Leitung (6) und die Fächer (3), (4) und (5) jeweils eine Tiefe (6a), (3a), (4a), (5a) in einem Bereich zwischen 0,4-0,9 mm aufweisen;
- sich das Fach (4) im gleichen Abstand zu den Endfächern (3) und (5) befindet;
- der Querschnitt, der durch die Ebene der Leitung (6) parallel zur Ebene der Oberfläche der Platte (1) definiert ist, eine Breite oder einen Durchmesser in einem Bereich zwischen 1 und 2 mm aufweist;
und
- die Platte eine Höhe oder Tiefe zwischen 0,5-20 mm aufweist und die Tiefe der Spur (2) in einem Bereich zwischen 20-80 % der Höhe oder Tiefe der Platte liegt.

2. Platte nach dem vorhergehenden Anspruch, umfassend mindestens 2 Spuren (2b) und (2a), die sich parallel zueinander in einem Abstand von mindestens 3 mm und im unteren Abschnitt der 2 Abschnitte der Platte befinden, die durch den Durchmesser oder die Längsachse (a) der Platte getrennt sind, wobei die Spuren (2a) und (2b) die gleiche Länge aufweisen.

3. Platte nach Anspruch 2, wobei der Trennabstand zwischen den Spuren 7 mm beträgt.

4. Platte nach einem der vorhergehenden Ansprüche, wobei der Abstand zwischen der Längsachse oder dem Durchmesser der Selektionsplatte (a) und der ihr am nächsten gelegenen Spur 7 mm beträgt.

5. Platte nach einem der vorhergehenden Ansprüche, wobei der Mikroinjektionsbereich (1) eine Vertiefung oder Rille zum Ablegen der Oozyten umfasst.

6. Platte nach einem der vorhergehenden Ansprüche, wobei die Platte kreisförmig oder oval ist,
und/oder
ferner einen Vorsprung (7) umfasst, der die Kontur umgibt.

7. Platte nach einem der vorhergehenden Ansprüche, wobei
- die Spur (2) eine Länge von 22 ± 0,2 mm aufweist,
- der Querschnitt, der durch die Ebene der Fächer (3) und (5) parallel zur Ebene der Oberfläche der Platte (1) definiert ist, eine Breite von 2,5 mm und eine Länge von 1,48 mm aufweist,
- der Querschnitt, der durch die Ebene des Fachs (4) parallel zur Ebene der Oberfläche der Platte (1) definiert ist, eine Breite von 5 mm und eine Länge von 3 mm aufweist;
- der Querschnitt, der durch die Ebene der Leitung (6) parallel zur Ebene der Oberfläche der Platte (1) definiert ist, eine Breite oder einen Durchmesser von 1 mm aufweist.

8. Verfahren zur physiologischen Selektion von Spermatozoen, umfassend die Verwendung der Selektionsplatte nach einem der Ansprüche 1-7 für eine verarbeitete Samenprobe,
umfassend die folgenden Schritte:
a) Bereitstellen von Cumulus-oophorus-Zellen im Fach (4) der Selektionsplatte nach einem der Ansprüche 1-7,
b) Bereitstellen einer verarbeiteten Samenprobe im Fach (3) der Selektionsplatte nach einem der Ansprüche 1-7,
c) Inkubieren der in Schritt a) und/oder b) erhaltenen Platte bei einer Temperatur in einem Bereich zwischen 36-38 °C für einen Zeitraum, der zwischen 10 Minuten und 3 Stunden umfasst, und
d) Extrahieren der Spermatozoen, die sich im Fach (5) befinden.

9. Verfahren nach dem vorhergehenden Anspruch, wobei die verarbeitete Samenprobe aus Schritt b) in einer Menge zwischen 1 und 10 µl und mit einer Konzentration zwischen 5 und 20 Millionen Spermatozoen/ml bereitgestellt wird.

10. Verfahren nach einem der vorhergehenden Ansprüche 8-9, wobei die Inkubationszeit von Schritt c) einen Bereich zwischen 30 min und 90 min umfasst.

11. Verfahren nach Anspruch 8, wobei:
i) die verarbeitete Samenprobe aus Schritt b) in einer Menge zwischen 2-5 µl und mit einer Konzentration von 10 Millionen Spermatozoen/ml bereitgestellt wird, und/oder
ii) die Inkubationszeit aus Schritt c) 60 Minuten beträgt.

12. Verfahren nach einem der vorhergehenden Ansprüche, wobei die Samenprobe menschlich ist.

13. Verwendung der Selektionsplatte nach einem der Ansprüche 1-7 in einem Verfahren zur Selektion von Spermatozoen.

## Revendications

1. Plaque de sélection physiologique de spermatozoïdes pour l'ICSI comprenant au moins deux parties, dans laquelle une partie comprend au moins une voie (2), chaque voie ayant trois compartiments (3, 4, 5) dans lesquels :
- le compartiment (3) est adapté pour recevoir un échantillon de sperme traité ;
- le compartiment (4) est adapté pour déposer les cellules de cumulus oophorus ;
- le compartiment (5) est adapté pour accueillir et récupérer les spermatozoïdes de l'échantillon de sperme traité ;
les compartiments (3) et (5) étant situés aux extrémités de la voie (2), le compartiment (4) étant situé entre les compartiments d'extrémité (3) et (5) et tous reliés au moyen d'un conduit (6) ;
**caractérisée en ce que**
- la voie (2) est configurée comme des renfoncements ou des rainures dans la plaque et sa longueur est dans une plage comprise entre 20 et 30 mm ;
- les compartiments (3), (4) et (5) et le conduit (6) sont formés au moyen d'un renfoncement ou d'une rainure dans la plaque ;
- la plaque comprend une zone de micro-injection (1) située dans la partie opposée à celle où se trouve la voie (2), ladite zone de micro-injection (1) étant adaptée au dépôt d'ovocytes et à la réalisation de la micro-injection des spermatozoïdes sélectionnés du compartiment (5) dans les ovocytes déposés, dans laquelle la zone de micro-injection (1) a une surface comprise entre 32 et 8 cm² ;
- la section définie par le plan des compartiments (3) et (5), parallèle au plan de la surface de la plaque, a une largeur ou un diamètre inférieur à la largeur ou au diamètre de la section définie par le plan du compartiment (4) parallèle au plan de la surface de la plaque, et qui se situe dans une plage comprise entre 2,5 et 4 mm, et
- le compartiment (4) a une surface plus grande que les compartiments (3) et (5) et que la section définie par le plan du compartiment (4), parallèle au plan de la surface de la plaque, a un diamètre ou une largeur compris entre 2,5 et 5 mm ;
- le conduit (6) et les compartiments (3), (4) et (5) ont une profondeur (6a), (3a), (4a), (5a) respectivement, dans une plage comprise entre 0,4 et 0,9 mm ;
- le compartiment (4) est situé à la même distance des compartiments d'extrémité (3) et (5) ;
- la section définie par le plan du conduit (6), parallèle au plan de la surface de la plaque (1), a une largeur ou un diamètre compris entre 1 et 2 mm ;
et
- la plaque a une hauteur ou une profondeur comprise entre 0,5 et 20 mm et la profondeur de la voie (2) est dans une plage comprise entre 20 et 80 % de la hauteur ou de la profondeur de la plaque.

2. Plaque selon la revendication précédente, comprenant au moins 2 voies (2b) et (2a) situées parallèles l'une à l'autre à une distance de séparation d'au moins 3 mm et dans la partie inférieure des 2 parties de la plaque divisées au moyen du diamètre ou de l'axe longitudinal (a) de la plaque, dans laquelle les voies (2a) et (2b) ont la même longueur.

3. Plaque selon la revendication 2, dans laquelle la distance de séparation entre les voies est de 7 mm.

4. Plaque selon l'une quelconque des revendications précédentes, la distance entre l'axe longitudinal ou le diamètre de la plaque de sélection (a) et la voie la plus proche de celle-ci est de 7 mm.

5. Plaque selon l'une quelconque des revendications précédentes, dans laquelle la zone de micro-injection (1) comprend un renfoncement ou une rainure pour déposer les ovocytes.

6. Plaque selon l'une quelconque des revendications précédentes, laquelle plaque est circulaire ou ovale,
et/ou
comprend en outre une protubérance (7) qui entoure le contour.

7. Plaque selon l'une quelconque des revendications précédentes, dans laquelle
- la voie (2) a une longueur de 22 ± 0,2 mm,
- la section définie par le plan des compartiments (3) et (5), parallèle au plan de la surface de la plaque (1), a une largeur de 2,5 mm et une longueur de 1,48 mm,
- la section définie par le plan du compartiment (4), parallèle au plan de la surface de la plaque (1), a une largeur de 5 mm et une longueur de 3 mm ;
- la section définie par le plan du conduit (6), parallèle au plan de la surface de la plaque (1), a une largeur ou un diamètre de 1 mm.

8. Procédé de sélection physiologique de spermatozoïdes comprenant l'utilisation de la plaque de sélection selon l'une quelconque des revendications 1 à 7, pour un échantillon de sperme traité,
comprenant les étapes suivantes consistant à :
a) la fourniture de cellules du cumulus oophorus dans le compartiment (4) de la plaque de sélection selon l'une quelconque des revendications 1 à 7,
b) la fourniture d'un échantillon de sperme traité dans le compartiment (3) de la plaque de sélection selon l'une quelconque des revendications 1 à 7,
c) l'incubation de la plaque obtenue à l'étape a) et/ou b) à une température dans une plage comprise entre 36 et 38 °C, pendant une période de temps comprise entre 10 minutes et 3 heures, et
d) l'extraction des spermatozoïdes qui se trouvent dans le compartiment (5).

9. Procédé selon la revendication précédente, dans lequel l'échantillon de sperme traité de l'étape b) est fourni en une quantité comprise entre 1 et 10 µl et ayant une concentration comprise entre 5 et 20 millions de spermatozoïdes/ml.

10. Procédé selon l'une quelconque des revendications 8 à 9, dans lequel la période d'incubation de l'étape c) est dans une plage comprise entre 30 min et 90 min.

11. Procédé selon la revendication 8, dans lequel :
i) l'échantillon de sperme traité de l'étape b) est fourni en une quantité comprise entre 2 et 5 µl et ayant une concentration de 10 millions de spermatozoïdes/ml, et/ou
ii) la période d'incubation de l'étape c) est de 60 minutes.

12. Procédé selon l'une quelconque des revendications précédentes, dans lequel l'échantillon de sperme est humain.

13. Utilisation de la plaque de sélection selon l'une quelconque des revendications 1 à 7 dans un procédé de sélection de spermatozoïdes.
